# EUROPEAN PATENT SPECIFICATION

(11) **EP 2 519 825 B1**
(45) Date of publication and mention of the grant of the patent: **14.01.2015**
(21) Application number: 10841723.9
(22) Date of filing: 30.12.2010
(51) Int. Cl.: G01N 33/574, C07H 21/04

(54) **SIMULTANEOUS DETECTION OF MUTATIONAL STATUS AND GENE COPY NUMBER**
GLEICHZEITIGE DETEKTION DES MUTATIONSSTATUS UND DER GENKOPIEZAHL
DÉTECTION SIMULTANÉE DE L'ÉTAT DE MUTATION ET DU NOMBRE DE COPIES D'UN GÈNE

(30) Priority: 31.12.2009 US 291444 P
(43) Date of publication of application: 07.11.2012
(73) Proprietor: Ventana Medical Systems, Inc., Tucson, Arizona 85755 (US)
(72) Inventor: SINGH, Shalini, Tucson Arizona 85750 (US); NITTA, Hiro, Oro Valley Arizona 85737 (US); GAIRE, Fabien, 82319 Starnberg (DE); DEL VALLE, Edmundo David, Tucson Arizona 85746 (US)
(74) Representative: Burger, Alexander
(86) International application number: PCT/US2010/062514
(87) International publication number: WO 2011/082307

(56) References cited:
- US-A1- 2008 090 233
- YU J ET AL: "381 POSTER Detecting EGFR mutations in NSCLC by mutant specific antibodies", EUROPEAN JOURNAL OF CANCER. SUPPLEMENT, PERGAMON, OXFORD, GB, vol. 6, no. 12, 1 October 2008 (2008-10-01), pages 119-120, XP025534445, ISSN: 1359-6349, DOI: 10.1016/S1359-6349(08)72315-2 [retrieved on 2008-10-01]
- L. V. SEQUIST ET AL: "First-Line Gefitinib in Patients With Advanced Non-Small-Cell Lung Cancer Harboring Somatic EGFR Mutations", JOURNAL OF CLINICAL ONCOLOGY, vol. 26, no. 15, 20 May 2008 (2008-05-20), pages 2442-2449, XP55018867, ISSN: 0732-183X, DOI: 10.1200/JCO.2007.14.8494
- YU ET AL.: 'Mutation-specific antibodies for the detection of EGFR mutations in non-small-cell lung cancer.' CLIN CAN RES 01 May 2009, pages 3023 - 3028, XP055008859
- VAN DER LOOS.: 'Multiple Immunoenzyme Staining: Methods and Visualizations for the Observation With Spectral Imaging.' J HISTOCHEM CYTOCHEM vol. 56, no. 4, April 2008, pages 313 - 328, XP055026599
- SHEPLEY ET AL.: 'Combined, Sequential In Situ Hybridization and Immunohistochemistry on the Same Tissue Section.' METH MOL BIO vol. 63, 1997, pages 247 - 256
- SHOLL ET AL.: 'Validation of chromogenic in situ hybridization for detection of EGFR copy number amplification in nonsmall cell lung carcinoma.' MOL PATHOL vol. 20, no. 10, October 2007, pages 1028 - 1035
- LI ALLAN R ET AL: "EGFR mutations in lung adenocarcinomas: clinical testing experience and relationship to EGFR gene copy number and immunohistochemical expression", JOURNAL OF MOLECULAR DIAGNOSTICS,THE, AMERICAN SOCIETY FOR INVESTIGATIVE PATHOLOGY, US, vol. 10, no. 3, 1 May 2008 (2008-05-01), pages 242-248, XP008161207, ISSN: 1525-1578, DOI: 10.2353/JMOLDX.2008.070178
- WILLMORE-PAYNE C ET AL: "Detection of epidermal growth factor receptor and human epidermal growth factor receptor 2 activating mutations in lung adenocarcinoma by high-resolution melting amplicon analysis: correlation with gene copy number, protein expression, and hormone receptor expression", HUMAN PATHOLOGY, SAUNDERS, PHILADELPHIA, PA, US, vol. 37, no. 6, 1 June 2006 (2006-06-01), pages 755-763, XP022759660, ISSN: 0046-8177, DOI: 10.1016/J.HUMPATH.2006.02.004 [retrieved on 2006-05-27]

## Description

### FIELD OF THE INVENTION

The present invention provides compositions and methods for simultaneously detecting mutational status and gene copy number. In particular, the present invention provides simultaneous measurement of EGFR gene copy number and detection of the L858R and Exon 19 del mutations of the EGFR gene in a tissue sample.

### BACKGROUND

Recognition that the epidermal growth factor receptor (EGFR) was a therapeutic target in non-small cell lung cancer (NSCLC) and other cancers has led to development of small molecule receptor tyrosine kinase inhibitors as cancer treatments. Clinical trials established that EGFR tyrosine kinase inhibitors produced objective responses in only a minority of NSCLC patients. It has been established that there is a significant correlation between EGFR gene copy number, and the presence of certain EGFR gene mutations, to the sensitivity of various NSCLC lines to EGFR tyrosine kinase inhibitors (Helfrich et al. Clin Cancer Res 2006;7117 12(23)). It has been demonstrated that EGFR gene copy amplification is focally distributed in lung cancer specimens, mostly in regions with solid histology, and that patients with EGFR amplification had Significantly worse outcomes (Sholl et al. Cancer Res 2009; 69: (21)). Lung adenocarcinomas with EGFR gene amplification and specific EGFR deletions and mutations show distinct clinicopathologic features associated with a significantly worsened prognosis.

Methods have been developed which are useful in detection of EGFR mutations in human tissues (Yu et al. Clin Cancer Res 3023 2009; 15(9)). The field currently lacks methods for simultaneous detection of EGFR gene copy number and EGFR mutational status at multiple loci. Such methods would provide significant advancement in diagnosing cancers associated with EGFR and determining effective treatment regimens for cancer patients. Additional technologies to address these and other deficiencies in the field are needed.

Carlynn Willmore-Payne et al (Human pathology, 2006) and Allan R. Li et al (Journal of molecular diagnostics, 2008) describe the detection of the EGFR status in tissue samples of cancer patients.

### SUMMARY OF THE INVENTION

The present invention provides compositions and methods for simultaneously detecting mutational status and gene copy number. In particular, the present invention provides simultaneous measurement of EGFR gene copy number and detection of the L858R and Exon 19 del mutations of the EGFR gene in tissue samples, for example from a cancer patient.

In some embodiments, the present invention provides a method for assessing the EGFR-status of a tissue sample as defined in claims 1-15.

In some embodiments, the present invention provides a kit as defined in claim 16.

### DESCRIPTION OF THE FIGURES

Figure 1 shows concurrent detection by L585R mutant specific IHC (Red), Exon 19 del specific IHC (blue), and EGFR gene ISH (black) of (A) wild-type EGFR, (B) Exon 19 del EGFR, and (C) L585R EGFR.
Figure 2 demonstrates the similar appearance between a silver *in situ* hybridization signal and anthracotic pigment.
Figure 3 shows concurrent detection by L585R mutant specific IHC (Blue), Exon 19 del specific IHC (Gold), and EGFR gene ISH (Red) of (A) wild-type EGFR, (B) Exon 19 del EGFR, and (C) L585R EGFR.
Figure 4 demonstrates the similar appearance between Red immunohistochemistry detection and red *in situ* hybridization detection.
Figure 5 shows concurrent detection by L585R mutant specific IHC (Red), Exon 19 del specific IHC (Gold), and EGFR gene ISH (Blue) of (A) wild-type EGFR, (B) Exon 19 del EGFR, and (C) L585R EGFR.
Figure 6 shows concurrent detection by L585R mutant specific IHC (Red), Exon 19 del specific IHC (Gold), and EGFR gene ISH (Blue) of (A) mutation negative EGFR, (B) Exon 19 del EGFR, and (C) L585R EGFR.
Figure 7 demonstrates the similar appearance between Red immunohistochemistry detection (A) and gold immunohistochemistry detection (B).
Figure 8 shows concurrent detection by L585R mutant specific and Exon 19 del specific IHC (Blue), and EGFR gene ISH (Red) of (A) wild-type EGFR, (B) Exon 19 del EGFR, and (C) L585R EGFR.
Figure 9 shows a comparison of IHC protein detection (A) and gene/protein dual detection (B).
Figure 10 shows a comparison of IHC protein detection (A) and gene/protein dual detection (B).
Figure 11 shows a comparison of IHC protein detection (A) and gene/protein dual detection (B).
Figure 12 shows a comparison of IHC protein detection (A) and gene/protein dual detection (B).
Figure 13 shows a comparison of IHC protein detection (A) and gene/protein dual detection (B).
Figure 14 shows concurrent gene/protein detection by L585R mutant specific and Exon 19 del specific IHC (Blue), and EGFR gene ISH (Red) of (A) mutation negative EGFR, (B) mutation mildly positive EGFR, and (C) mutation positive EGFR.
Figure 15 shows tumor heterogeneity using concurrent gene/protein detection by L585R mutant specific and Exon 19 del specific IHC (Blue), and EGFR gene ISH (Red).
Figure 16 shows tumor heterogeneity using concurrent gene/protein detection by L585R mutant specific and Exon 19 del specific IHC (Blue), and EGFR gene ISH (Red).

### DEFINITIONS

### To facilitate an understanding of the present invention, a number of terms and phrases are defined below:

As used herein, the term "copy number" or "gene copy number" as used in reference to specific nucleic acid sequences (e.g. EGFR, wild-type EGFR, EGFR Exon 19 del, EGFR L858R and control) refers to the actual number of these sequences per single cell. Copy number may be reported for one single cell, or reported as the average number in a group of cells (e.g., tissue sample). When comparing the "copy number" of cells (e.g., experimental and control cells) one need not determine the exact copy number of the cell, but instead need only obtain an approximation that allows one to determine whether a given cell contains more or less of the nucleic acid sequence as compared to another cell. Thus, any method capable of reliably directly or indirectly determining amounts of nucleic acid may be used as a measure of copy number even if the actual copy number is not determined.

As used herein, the term "amplification" when used in reference to copy number refers to the condition in which the copy number of a nucleic acid sequence *(e.g.,* EGFR, wild-type EGFR, EGFR Exon 19 del, EGFR L858R) is greater than the copy number of a control sequence (*e.g.,* chromosome 17). In other words, amplification indicates that the ratio of a particular nucleic acid sequence (*e.g.,* EGFR, wild-type EGFR, EGFR Exon 19 del, EGFR L858R) is greater than 1:1 when compared to a control sequence (*e.g.,* 1.1:1, 1.2:1, or 1.3:1). In preferred embodiments, the ratio of a particular nucleic acid sequence is at least 1.5 times greater than the control sequence copy number (*i.e.,* 1.5:1).

As used herein, the term "nucleic acid molecule" and "nucleic acid sequence" refer to any nucleic acid containing molecule including, but not limited to DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As used herein, the term "hybridization" is used in reference to the pairing of complementary nucleic acids. Hybridization and the strength of hybridization (e.g., the strength of the association between the nucleic acids) is impacted by such factors as the degree of complementary between the nucleic acids, stringency of the conditions involved, the Tₘ of the formed hybrid, and the G:C ratio within the nucleic acids.

As used herein, the term "probe" refers to an oligonucleotide (*i.e.,* a sequence of nucleotides), or a library of nucleotide fragments, whether occurring naturally as in a purified restriction digest or produced synthetically, recombinantly or by amplification (e.g. PCR), which is capable of hybridizing to an oligonucleotide of interest. Probes useful in the present invention may be single-stranded or doublestranded. Probes are useful in the detection, identification and isolation of particular gene sequences (*e.g.,* EGFR, wild-type EGFR, EGFR Exon 19 del, EGFR L858R). It is contemplated that any probe used in the present invention may be labeled with any "reporter molecule" or detectable substrate, so it is detectable in any detection system, including, but not limited to enzyme (*e.g.,* ELISA, as well as enzyme-based immunohistochemical assays), fluorescent (*e.g.,* FISH), chromogenic (e.g. CISH), radioactive, mass spectroscopy, and luminescent systems. It is not intended that the present invention be limited to any particular detection system or label.

As used herein, the term "label" refers to any molecule which may be detected. For example, labels include, but are not limited to, ³²P, ¹⁴C, ¹²⁵I, ³H, ³⁵S, biotin, digoxigenin (DIG), dinitrophenol (DNP), avidin, chromogenic, fluorescent or enzymatic molecules.

As used herein, the terms *"in situ* hybridization" and "ISH" refer to methods for detecting and localizing nucleic acids within a cell or tissue preparation. These methods provide both quantitative and spatial information concerning the nucleic acid sequences within an individual cell or chromosome. ISH has been commonly used in many areas, including prenatal genetic disorder diagnosis, molecular cytogenetics, to detect gene expression and overexpression, to identify sites of gene expression, to map genes, to localize target genes and to identify various viral and microbial infections, tumor diagnosis, *in vitro* fertilization analysis, analysis of bone marrow transplantation and chromosome analysis. The technique generally involves the use of labeled nucleic acid probes which are hybridized to a chromosome or mRNA in cells that are mounted on a surface (e.g slides or other material). The probes can be labeled with fluorescent molecules, dyes, or other labels. One example of fluorescent *in situ* hybridization (FISH) is provided in Kuo et al., Am. J. Hum. Genet., 49:112-119, 1991. Other ISH and FISH detection methods are provided in U.S. Pat., 5,750,340 to Kim et al.*.* An example of chromogenic *in situ* hybridization (CISH) is provided in U.S. Pat., 6,942,970 to Isola et al.. A further example of CISH is *silver in situ* hybridization (SISH) as provided in US Patent Nos. 6,670,113, 7,183,072, 7,364,484, 7,592,153, 7,632,652 and 7,642,064, and US Patent Publication Nos. 2007/0224625 and 2008/0213783. Additional protocols are known to those of skill in the art.

As used herein, the phrase "under *in situ* hybridization conditions" refers to any set of conditions used for performing *in situ* hybridization (ISH) that allows the successful detection of labeled oligonucleotide probes. Generally, the conditions used for *in situ* hybridization involve the fixation of tissue or other biological sample onto a surface, prehybridization treatment to increase the accessibility of target nucleic acid sequences in the sample (and to reduce non-specific binding), hybridization of the labeled nucleic acid probes to the target nucleic acid, post-hybridization washes to remove unbound probe, and detection of the hybridized probes. Each of these steps is well known in the art and has been performed under many different experimental conditions. Again, examples of such *in situ* hybridization conditions are provided in Kuo et al., U.S. Pat. 5,750,340, and U.S. Pat., 6,942,970 to Isola et al. Further examples of conditions and reagents are provided below.

The tissue or biological sample can be fixed to a surface using fixatives. Preferred fixatives cause fixation of the cellular constituents through a precipitating action which is reversible, maintains a cellular morphology with the nucleic acid in the appropriate cellular location, and does not interfere with nucleic acid hybridization. Examples of fixatives include, but are not limited to, formaldehyde, alcohols, salt solutions, mercuric chloride, sodium chloride, sodium sulfate, potassium dichromate, potassium phosphate, ammonium bromide, calcium chloride, sodium acetate, lithium chloride, cesium acetate, calcium or magnesium acetate, potassium nitrate, potassium dichromate, sodium chromate, potassium iodide, sodium iodate, sodium thiosulfate, picric acid, acetic acid, sodium hydroxide, acetones, chloroform glycerin, and thymol.

After being fixed on a surface, the samples are treated to remove proteins and other cellular material which may cause nonspecific background binding. Agents which remove protein include, but are not limited to, enzymes such as pronase and proteinase K, or mild acids, such as 0.02.-0.2 HCl, as well as RNase (to remove RNA).

DNA on the surface is denatured so that the oligonucleotide probes can bind. Denaturation can be accomplished, for example, by varying the pH, increasing temperature, or with organic solvents such as formamide. The labeled probe may then hybridize with the denatured DNA under standard hybridization conditions. The tissue or biological sample may be deposited on a solid surface using standard techniques such as sectioning of tissues or smearing or cytocentrifugation of single cell suspensions. Examples of solid surfaces include, but are not limited to, glass, nitrocellulose, adhesive tape, nylon, or GENE SCREEN PLUS.

As used herein, the terms "anticancer agent," "conventional anticancer agent," or "cancer therapeutic drug" refer to any therapeutic agents (e.g., chemotherapeutic compounds and/or molecular therapeutic compounds), radiation therapies, or surgical interventions, used in the treatment of cancer (e.g., in mammals, in primates, in humans, etc.).

As used herein, the terms "drug" and "chemotherapeutic agent" refer to pharmacologically active molecules that are used to diagnose, treat, or prevent diseases or pathological conditions in a physiological system *(e.g.,* a subject, or *in vivo, in vitro,* or *ex vivo* cells, tissues, and organs). Drugs act by altering the physiology of a living organism, tissue, cell, or *in vitro* system to which the drug has been administered. It is intended that the terms "drug" and "chemotherapeutic agent" encompass anti-hyperproliferative and antineoplastic compounds as well as other biologically therapeutic compounds.

An "effective amount" is an amount sufficient to effect beneficial or desired results. An effective amount can be administered in one or more administrations.

As used herein, the term "administration" refers to the act of giving a drug, prodrug, antibody, or other agent, or therapeutic treatment to a physiological system *(e.g.,* a subject or *in vivo, in vitro,* or *ex vivo* cells, tissues, and organs). Exemplary routes of administration to the human body can be through the eyes (ophthalmic), mouth (oral), skin (transdermal), nose (nasal), lungs (inhalant), oral mucosa (buccal), ear, by injection (*e.g.,* intravenously, subcutaneously, intratumorally, intraperitoneally, *etc.*) and the like.

"Coadministration" refers to administration of more than one chemical agent or therapeutic treatment *(e.g.,* radiation therapy) to a physiological system *(e.g.,* a subject or *in vivo, in vitro,* or *ex vivo* cells, tissues, and organs). "Coadministration" of the respective chemical agents may be concurrent, or in any temporal order or physical combination. "Coadministration" of therapies is a treatment course of action that is called upon for many types of cancer.

As used herein, the term "subject" refers to organisms subjected to the compositions and methods of the present invention. Such organisms include, but are not limited to, humans, non-human primates, dogs, cats, horses, pigs, cattle, sheep, goats, mice, rats, and the like. In some embodiments, tissue, organs, fluids (e.g. blood, urine, saliva, etc.), samples, etc. may be taken from a "subject" and used in conjunction with the present invention.

As used herein, the term "patient" generally refers to a subject who will receive or who has received treatment for a disease or condition, or who is being screened for a disease or condition.

The term "diagnosed," as used herein, refers to the recognition of a disease by its signs and symptoms or genetic analysis, pathological analysis, histological analysis, and the like. A subject or patient is "diagnosed" with cancer when cancer is identified in a subject by any suitable means. A subject "diagnosed" with cancer may or may not exhibit any signs or symptoms of the cancer.

As used herein, a "subject suspected of having cancer" is any subject or patient to be tested or assayed by any suitable method, including embodiments of the present invention, for the presence of cancer, cancer cells, tumors, pre-cancerous cells, etc. The subject may or may not have risk factors for cancer, and may or may not exhibit any signs or symptoms of cancer.

As used herein, a "subject suffering from cancer" is any subject or patient who exhibits signs or symptoms of cancer. A "subject suffering from cancer" may or may not have received any cancer testing, and may or may not have received a cancer diagnosis.

As used herein, the term "antigen binding protein" refers to proteins which bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibodies, various host animals can be immunized by injection with the peptide corresponding to the desired epitope including, but not limited to, rabbits, mice, rats, sheep, goats, *etc.* In a preferred embodiment, the peptide is conjugated to an immunogenic carrier (*e.g.,* diphtheria toxoid, bovine serum albumin (BSA), or keyhole limpet hemocyanin (KLH)). Various adjuvants are used to increase the immunological response, depending on the host species, including, but not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

For preparation of monoclonal antibodies, any technique that provides for the production of antibody molecules by continuous cell lines in culture may be used (*See e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 (1975)), as well as the trioma technique, the human B-cell hybridoma technique (*See e.g.,* Kozbor et al., Immunol. Today, 4:72 (1983)), and the EBV-hybridoma technique to produce human monoclonal antibodies (Cole et al., in Monoclonal Antibodies and Cancer Therapy, Alan R. Liss, Inc., pp. 77-96 (1985)).

According to the invention, techniques described for the production of single chain antibodies (U.S. 4,946,778) can be adapted to produce specific single chain antibodies as desired. An additional embodiment of the invention utilizes the techniques known in the art for the construction of Fab expression libraries (Huse et al., Science, 246:1275-1281 (1989)) to allow rapid and easy identification of monoclonal Fab fragments with the desired specificity.

Antibody fragments that contain the idiotype (antigen binding region) of the antibody molecule can be generated by known techniques. For example, such fragments include, but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen-binding proteins can be isolated by methods known in the art. In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g.,* gel agglutination assays, hemagglutination assays, *etc.*), complement fixation assays, immunofluorescence assays, protein A assays, and immunoelectrophoresis assays, *etc*.) *etc.*

The term "antibody" is used in the broadest sense and specifically covers monoclonal antibodies, polyclonal antibodies, multispecific antibodies (e.g., bispecific antibodies), and antibody fragments so long as they bind specifically to a target antigen.

The term "primary antibody" herein refers to an antibody which binds specifically to the target protein antigen in a tissue sample. A primary antibody is generally the first antibody used in an immunohistochemical procedure. In one embodiment, the primary antibody is the only antibody used in an IHC procedure. With regards to ISH, a primary antibody is typically directed to a label, wherein said label (e.g., hapten, etc.) is incorporated into an oligonucleotide probe that is directed to a target sequence. For example, if an oligonucleotide probe is labeled with DIG the primary antibody is an anti-DIG antibody that recognizes and binds the DIG hapten of the oligonucleotide. Further, such a primary antibody used in ISH is typically conjugated to a detection reagent or enzyme which provides a detection means for detecting hybridization events.

The term "secondary antibody" herein refers to an antibody which binds specifically to a primary antibody, thereby forming a bridge between the primary antibody and a subsequent reagent, if any. The secondary antibody is generally the second antibody used in an immunohistochemical procedure.

The term "stain" used as a noun or the term "staining reagent" refers to biological or chemical compounds, and compositions containing such compounds which, when applied to targeted molecules in biological sample, renders the molecules detectable (e.g. under microscopic examination). Stains include without limitation detectable nucleic acid probes, antibodies, and other reagents which in combination or by themselves result in a colored end product. The term "stain" is used interchangeably with the term "dye." The term "stain" used as a verb, or the term "staining," means the contacting of a staining reagent, stain, or dye with a biological sample.

As used herein, "tissue sample" is meant a collection of similar cells obtained from a tissue of a subject or patient, preferably containing genetic and/or chromosomal material. The four main human tissues are (1) epithelium; (2) the connective tissues, including blood vessels, bone and cartilage; (3) muscle tissue; and (4) nerve tissue. The source of the tissue sample may be solid tissue as from a fresh, frozen, and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. The tissue sample may contain compounds which are not naturally intermixed with the tissue in nature such as preservatives, anticoagulants, buffers, fixatives, nutrients, antibiotics, or the like.

For the purposes herein, a "section" of a tissue sample is a single part or piece of a tissue sample, e.g. a thin slice of tissue or cells cut from a tissue sample. It is understood that multiple sections of tissue samples may be taken and subjected to analysis according to the present invention.

### DETAILED DESCRIPTION OF THE INVENTION

The present invention provides methods and kits for simultaneously detecting mutational status and gene copy number as defined in claims 1-16. In particular, the present invention provides simultaneous measurement of gene copy number, L858R mutation status and Exon 19 del mutation status in tissue samples from a patient. The information derived from these measurements can then be used to make a diagnosis and/or prognosis for the patient and to determine a therapeutic treatment for the patient.

### 1. Reagents

The methods and kits of claims 1-16 of the present invention utilize a variety of reagents, including but not limited to, antigen binding molecules, nucleic acid probes, detectable moieties, signal generating moieties and signal generating systems. These reagents are used to generate detectable signals so that the presence or absence of EGFR mutations and EGFR gene amplification can be detected.

### Antigen Binding Molecules

In some embodiments, the present invention utilizes antigen binding molecules to detect EGFR mutations. In some embodiments, the present invention provides reagents for the specific detection of EGFR Exon 19 del and/or EGFR L858R. In some embodiments, the present invention provides antigen-binding proteins (e.g. antibodies) specific to EGFR Exon 19 del. In some embodiments, the present invention provides antigen-binding proteins (e.g. antibodies) specific to EGFR L858R. In some embodiments, EGFR Exon 19 del and EGFR L858R are detected in a sample (e.g. cells, cell culture, tissue sample, tumor sample, etc.) using reagents (e.g. antigen-binding proteins (e.g. antibodies)) of the present invention. In some embodiments, antibodies are used to detect, identify, and/or quantify EGFR Exon 19 del and/or EGFR L858R in a tissue sample from a subject suspected of having cancer, a subject diagnosed with cancer, and/or a subject suffering from cancer. In some embodiments, EGFR Exon 19 del and EGFR L858R are detected concurrently with other forms (e.g. wild-type, mutant) of EGFR. In some embodiments, EGFR Exon 19 del and EGFR L858R are detected concurrently with one or more references or standards (e.g. another gene, wild-type EGFR, etc.).

The present disclosure provides isolated antibodies and antibody fragments against EGFR, wild-type EGFR, EGFR Exon 19 del, EGFR L858R, and other variants of EGFR. The antibody, or antibody fragment, can be any monoclonal or polyclonal antibody that specifically recognizes these proteins. In some embodiments, the present disclosure provides monoclonal antibodies, or fragments thereof, specific to EGFR, wild-type EGFR, EGFR Exon 19 del, EGFR L858R, and other variants of EGFR. In some embodiments, the monoclonal antibodies, or fragments thereof, are chimeric or humanized antibodies that specifically bind to these proteins. In other embodiments, the monoclonal antibodies, or fragments thereof, are human antibodies that specifically bind to these proteins.

The antibodies against EGFR, wild-type EGFR, EGFR Exon 19 del, EGFR L858R, and other variants of EGFR find use in the experimental, diagnostic and therapeutic methods described herein. In certain embodiments, the antibodies of the present disclosure are used to detect the expression of EGFR, wild-type EGFR, EGFR Exon 19 del, EGFR L858R, and other variants of EGFR in biological samples such as, for example, a patient tissue biopsy or blood sample.

In some preferred embodiments, the antibodies specific for EGFR mutations are obtained from Cell Signaling Technologies, Danvers, MA. In general, polyclonal antibodies can be prepared by any known method. Polyclonal antibodies can be raised by immunizing an animal (e.g. a rabbit, rat, mouse, donkey, etc.) by multiple subcutaneous or intraperitoneal injections of the relevant antigen (a purified peptide fragment, full-length recombinant protein, fusion protein, etc.)(e.g. EGFR, wild-type EGFR, EGFR Exon 19 del, EGFR L858R, and other variants of EGFR) optionally conjugated to keyhole limpet hemocyanin (KLH), serum albumin, etc. diluted in sterile saline and combined with an adjuvant (e.g. Complete or Incomplete Freund's Adjuvant) to form a stable emulsion. The polyclonal antibody is then recovered from blood, ascites and the like, of an animal so immunized. Collected blood is clotted, and the serum decanted, clarified by centrifugation, and assayed for antibody titer. The polyclonal antibodies can be purified from serum or ascites according to standard methods in the art including affinity chromatography, ion-exchange chromatography, gel electrophoresis, dialysis, etc.

Monoclonal antibodies can be prepared using hybridoma methods, such as those described by Kohler and Milstein (1975) Nature 256:495. Using the hybridoma method, a mouse, hamster, or other appropriate host animal, is immunized as described above to elicit the production by lymphocytes of antibodies that will specifically bind to an immunizing antigen (EGFR, wild-type EGFR, EGFR Exon 19 del, EGFR L858R, and other variants of EGFR). Alternatively, lymphocytes can be immunized in vitro. Following immunization, the lymphocytes are isolated and fused with a suitable myeloma cell line using, for example, polyethylene glycol, to form hybridoma cells that can then be selected away from unfused lymphocytes and myeloma cells. Hybridomas that produce monoclonal antibodies directed specifically against a chosen antigen (e.g. EGFR, wild-type EGFR, EGFR Exon 19 del, EGFR L858R, and other variants of EGFR) as determined by immunoprecipitation, immunoblotting, or by an in vitro binding assay such as radioimmunoassay (RIA) or enzyme-linked immunosorbent assay (ELISA) can then be propagated either in vitro culture using standard methods (Goding, Monoclonal Antibodies: Principles and Practice, Academic Press, 1986) or in vivo as ascites tumors in an animal. The monoclonal antibodies can then be purified from the culture medium or ascites fluid as described for polyclonal antibodies above.

Alternatively monoclonal antibodies can also be made using recombinant DNA methods as described in U.S. Patent 4,816,567. The polynucleotides encoding a monoclonal antibody are isolated, such as from mature B-cells or hybridoma cell, such as by RT-PCR using oligonucleotide primers that specifically amplify the genes encoding the heavy and light chains of the antibody, and their sequence is determined using conventional procedures. The isolated polynucleotides encoding the heavy and light chains are then cloned into suitable expression vectors, which when transfected into host cells such as E. coli cells, simian COS cells, Chinese hamster ovary (CHO) cells, or myeloma cells that do not otherwise produce immunoglobulin protein, monoclonal antibodies are generated by the host cells. Also, recombinant monoclonal antibodies or fragments thereof of the desired species can be isolated from phage display libraries as described (McCafferty et al., 1990, Nature, 348:552-554; Clackson et al., 1991, Nature, 352:624-628; and Marks et al., 1991, J. Mol. Biol., 222:581-597).

The polynucleotide(s) encoding a monoclonal antibody can further be modified in a number of different manners using recombinant DNA technology to generate alternative antibodies. In one embodiment, the constant domains of the light and heavy chains of, for example, a mouse monoclonal antibody can be substituted 1) for those regions of, for example, a human antibody to generate a chimeric antibody or 2) for a non-immunoglobulin polypeptide to generate a fusion antibody. In other embodiments, the constant regions are truncated or removed to generate the desired antibody fragment of a monoclonal antibody. Furthermore, site-directed or high-density mutagenesis of the variable region can be used to optimize specificity, affinity, etc. of a monoclonal antibody.

In some embodiments of the present disclosure, the monoclonal antibody against EGFR, wild-type EGFR, EGFR Exon 19 del, EGFR L858R, and/or other variants of EGFR is a humanized antibody.

This disclosure also encompasses bispecific antibodies that specifically recognize EGFR, wild-type EGFR, EGFR Exon 19 del, EGFR L858R, and other variants of EGFR. Bispecific antibodies are antibodies that are capable of specifically recognizing and binding at least two different epitopes.

Bispecific antibodies can be intact antibodies or antibody fragments. Techniques for making bispecific antibodies are common in the art (Millstein et al., 1983, Nature 305:537-539; Brennan et al., 1985, Science 229:81; Suresh et al, 1986, Methods in Enzymol. 121:120; Traunecker et al., 1991, EMBO J. 10:3655-3659; Shalaby et al., 1992, J. Exp. Med. 175:217-225; Kostelny et al., 1992, J. Immunol. 148:1547-1553; Gruber et al., 1994, J. Immunol. 152:5368; and U.S. Patent 5,731,168).

In certain embodiments of the invention, it may be desirable to use an antibody fragment, rather than an intact antibody, to increase tissue penetration, for example. Various techniques are known for the production of antibody fragments. Traditionally, these fragments are derived via proteolytic digestion of intact antibodies (for example Morimoto et al., 1993, Journal of Biochemical and Biophysical Methods 24:107-117 and Brennan et al., 1985, Science, 229:81). However, these fragments are now typically produced directly by recombinant host cells as described above. Thus Fab, Fv, and scFv antibody fragments can all be expressed in and secreted from E. coli or other host cells, thus allowing the production of large amounts of these fragments. Alternatively, such antibody fragments can be isolated from antibody phage libraries. The antibody fragment can also be linear antibodies as described in U.S. Patent 5,641,870, for example, and can be monospecific or bispecific. Other techniques for the production of antibody fragments will be apparent to th skilled practitioner.

The present disclosure further embraces variants and equivalents which are substantially homologous to the chimeric, humanized and human antibodies, or antibody fragments thereof, set forth herein. These can contain, for example, conservative substitution mutations, i.e. the substitution of one or more amino acids by similar amino acids. For example, conservative substitution refers to the substitution of an amino acid with another within the same general class such as, for example, one acidic amino acid with another acidic amino acid, one basic amino acid with another basic amino acid or one neutral amino acid by another neutral amino acid. What is intended by a conservative amino acid substitution is well known in the art.

In some embodiments, the antibodies described above are labeled with a detectable moiety that produces a detectable signal or which is part of a signal generating system. Detectable moieties are described in more detail below and include haptens, fluorescent molecules, enzymes, luminescent molecules, quantum dots, and the like. In some embodiments, direct detection is utilized when the detectable moiety is for example, a fluorescent molecule, enzyme, luminescent molecule, or quantum dots. In some embodiments, e.g., where the detectable moiety is a hapten, indirect detection of the hapten-labeled primary antibody (e.g., the antibody specific for a particular EGFR mutation) via a signal generating system is utilized. The skilled artisan will be aware of various techniques for achieving this. In some embodiments, the signal generating system comprises at least first and second members of a binding pair. The first member of the binding pair is conjugated to the primary antibody and the second member of the binding pair is used to detect the first member. In some embodiments, the second member is labeled with a signal generating moiety. In other embodiments, a third member that binds to the second member is utilized for detection. In these embodiments, the third member comprises a signal generating moiety. For example, the antibody can be conjugated with a hapten and any of the four broad categories of labels mentioned above can be conjugated with an anti-hapten antibody (e.g., a secondary antibody) to achieve detection. When an enzymatic label is used, substrates for the enzyme are contacted with the sample to produce a detectable signal. Alternatively, in some embodiments, the secondary antibody is not labeled. In these embodiments, a tertiary antibody that is specific for the secondary antibody is utilized. For example, if the secondary antibody is a rabbit antibody, the tertiary antibody can be an anti-rabbit antibody conjugated to one of the labels described above. Thus, indirect conjugation of the label with the antibody can be achieved.

### 2. Nucleic Acid Probes

The present invention utilizes nucleic acid probes. In preferred embodiments, the nucleic acid probes bind or hybridize to portions of a target nucleic acid sequence (e.g., the sequence for EGFR). Preferably, the nucleic acid probe comprises any suitable nucleic acid, such as RNA (Ribonucleic acid), DNA (Deoxyribonucleic acid), LNA (Locked Nucleic Acid), PNA (Peptide Nucleic Acid) or combinations thereof, and can comprise both standard nucleotides such as ribonucleotides and deoxyribonucleotides and nucleotide analogs.

In some embodiments, the nucleic acid probe is greater than 80% complementary to the desired portion of the target nucleic acid sequence (e.g., the sequence for EGFR), preferably greater than 90% complementary to the desired portion of the target nucleic acid sequence, more preferably greater than 99% complementary to the desired portion of the target nucleic acid sequence, and most preferably about 100% complementary to the desired portion of the target nucleic acid sequence. In general, design of the nucleic acid probe is accomplished using practices that are standard in the art. For example, sequences that have self-complementarity, such that the resulting probes would either fold upon themselves, or hybridize to each other at the expense of binding to the target nucleic acid, are generally avoided.

One consideration in choosing a length for the target probe portion is the complexity of the sample containing the target nucleic acid. For example, the human genome is approximately 3X10⁹ base pairs in length. Any 10-nucleotide sequence will appear with a frequency of approximately 2,861 times in 3 billion base pairs. A target probe portion of this length would have a poor chance of binding uniquely to a 10 nucleotide region within a target having a sequence the size of the human genome. If the target sequence were within a 3 kb plasmid, however, such an oligonucleotide might have a very reasonable chance of binding uniquely. By this same calculation it can be seen that an oligonucleotide of 16 nucleotides (i.e., a 16-mer) is the minimum length of a sequence that is mathematically likely to appear once in 3X10⁹ base pairs. This level of specificity may also be provided by two or more shorter nucleic acid sequences if they are configured to bind in a cooperative fashion (i.e., such that they can produce the intended complex only if both or all are bound to their intended target sequences), wherein the combination of the short sequences provides the desired specificity.

A second consideration in choosing target probe portion length is the temperature range in which the target probe portion will be expected to function. A 16-mer of average base content (50% G-C bases) will have a calculated Tₘ of about 41°C, depending on, among other things, the concentration of the probe and its target, the salt content of the reaction and the precise order of the nucleotides. As a practical matter, longer target probe portions are usually chosen to enhance the specificity of hybridization. For example, target probe portions of from 20 to 25 nucleotides in length can be used, as they are highly likely to be specific if used in reactions conducted at temperatures which are near their Tₘs (within about 5°C of the Tₘ).

In preferred embodiments, the nucleic acid probe is designed taking these considerations into account, so that the target probe portion will hybridize to a target nucleic acid under suitable conditions defined by the user.

The nucleic acid can be selected manually, or with the assistance of a computer implemented algorithm that optimizes primer selection based on desired parameters, such as temperature, length, GC content, etc. Numerous computer implemented algorithms or programs for use via the internet or on a personal computer are available. For example, to generate multiple binding regions from a target nucleic acid sequence (e.g., genomic target nucleic acid sequence), regions of sequence devoid of repetitive (or other undesirable, e.g., background-producing) nucleic acid sequence are identified, for example manually or by using a computer algorithm. Within a target nucleic acid sequence (e.g., genomic target nucleic acid sequence) that spans several to several-hundred kilobases, typically numerous binding regions that are substantially or completely free of repetitive (or other undesirable, e.g., background-producing) nucleic acid sequences are identified.

The nucleic acid probes can be synthesized by any known method. In some embodiments, the sequences encoding the nucleic acid probes are cloned into a plasmid expression vector. The nucleic probe is preferably transcribed from the vector with an RNA polymerase to provide an RNA molecule encoding the nucleic acid probe. In some embodiments, the nucleic acid probe is chemically synthesized, for example, using phosphoramidite analogs. In some embodiments, DNA probes are synthesized by propagation, purification and restriction digestion of plasmid DNA to provide a DNA molecule encoding the target nucleic acid probe. The double stranded DNA can be subsequently melted into single strands for use in hybridization protocols. In some embodiments, the target nucleic acid probes are synthesized by asymmetric PCR. In some embodiments, one primer, could for example, be a nucleic acid analog (e.g., LNA). This process generates a probe with the target specific portion containing locked nucleotides and the detection target portion being made from standard dNTP's. In some embodiments, the LNA containing primer contains a biotin to facilitate purification of the desired strand.

In some embodiment, the nucleic acid probes comprise a one or detectable moieties. In some embodiments, the detectable moieties are directly detectable, while in other embodiments, the detectable moieties are indirectly detectable. In some embodiments, the detectable moieties are signal generating moieties that produce a detectable signal. In some embodiments, the detectable moiety is conjugated to nucleotides or nucleotide analogs used in the synthesis of the detection probe. For example, nucleoside phosphoramidites that are conjugated to a desired detectable moiety are used to synthesize a detection probe via chemical synthesis as is known in the art.

In some embodiments, the detectable moiety is detected indirectly. In some embodiments, the detectable moiety is a first member of a signal generating system. In these embodiments, nucleotides conjugated to a first member of a binding pair are incorporated into the detection probe, preferably via the use nucleoside phosphoramidites conjugated to the first member of the binding pair. The sample is then contacted with a specific binding agent comprising the second member of the binding pair (i.e., a specific binding moiety). In some embodiments, the second member of the binding pair is conjugated to a signal generating moiety and used detect the detection probe via binding to the first member of the binding pair. In other embodiments, the sample is contacted with a third binding member which binds to the second binding member. In these embodiments, the third binding member is conjugated to a signal generating moiety. Examples of suitable binding molecule systems include, but are not limited to, avidin, biotin, haptens, anti-hapten antibodies, and anti-antibody antibodies, and combinations thereof. For example, in some embodiments, the detectable moiety portion of the detection probe comprises a one or more haptenylated nucleotides. These haptenylated nucleotides are detected by the use of an antihapten antibody and an anti-(antihapten antibody) antibody that is conjugated to a signal generating moiety.

### Signal Generating Systems

As described above, the systems and methods of the present invention utilize a variety of reagents, for example, antigen binding molecules and nucleic acid probes that are labeled with detectable moieties for direct detection (i.e., labeled with signal grating moieties) or for indirect detection (i.e., labeled with detectable moiety that is a first member of a binding pair or signal generating system. The present invention is not limited to any particular label or signal. Indeed, a variety of signals and signal generating systems can be used in the described systems and methods, including, but not limited to, colorimetric signals, fluorescent signals, luminescent signals, and the like. In some preferred embodiments, colorimetric signals are used. In some preferred embodiments, the primary antibodies and nucleic acid probes are detected by indirect detection via the use of haptenylated primary antibodies and nucleic acid probes. In some further preferred embodiments, the signals are compatible with one another so that multiple signals (e.g., signals for the two EGFR mutations and EGFR amplification) can be visualized simultaneously on a sample from a patient.

Accordingly, in some embodiments, the present disclosure provides signal generating systems which utilize haptenylated primary antibodies and nucleic acid probes. Such haptens include, but are not limited to, pyrazoles, particularly nitropyrazoles; nitrophenyl compounds; benzofurazans; triterpenes; ureas and thioureas, particularly phenyl ureas, and even more particularly phenyl thioureas; rotenone and rotenone derivatives, also referred to herein as rotenoids; oxazole and thiazoles, particularly oxazole and thiazole sulfonamides; coumarin and coumarin derivatives; cyclolignans, exemplified by Podophyllotoxin and Podophyllotoxin derivatives; and combinations thereof. Specific examples of haptens include, but are not limited to, 2,4-Dintropheyl (DNP), Biotin, Fluorescein deratives (FITC, TAMRA, Texas Red, etc.), Digoxygenin (DIG), 5-Nitro-3-pyrozolecarbamide (nitropyrazole, NP), 4,5,-Dimethoxy-2-nitrocinnamide (nitrocinnamide, NCA), 2-(3,4-Dimethoxyphenyl)-quinoline-4-carbamide (phenylquinolone, DPQ), 2,1,3-Benzoxadiazole-5-carbamide (benzofurazan, BF), 3-Hydroxy-2-quinoxalinecarbamide (hydroxyquinoxaline, HQ), 4-(Dimethylamino)azobenzene-4'-sulfonamide (DABSYL), Rotenone isoxazoline (Rot), (E)-2-(2-(2-oxo-2,3-dihydro-1H-benzo[b][1,4]diazepin-4-yl)phenozy)acetamide (benzodiazepine, BD), 7-(diethylamino)-2-oxo-2H-chromene-3-carboxylic acid (coumarin 343, CDO), 2-Acetamido-4-methyl-5-thiazolesulfonamide (thiazolesulfonamide, TS), and p-Mehtoxyphenylpyrazopodophyllamide (Podo). These haptens and their use in probes are described in more detail in co-owned applications US Pat. Publ. Nos. 20080305497, 20080268462, and 20080057513.

In embodiments where the primary antibody or nucleic acid probe comprises haptens, the second member of the signal generating system is preferably a molecule that binds to the hapten such as an antigen binding molecule. Examples of suitable antigen binding molecules include, but are not limited to, antibodies, immunoglobulins or immunoglobulin-like molecules (including by way of example and without limitation, IgA, IgD, IgE, IgG and IgM), antibody fragments such as F(ab')₂ fragments, Fab' fragments, Fab'-SH fragments and Fab fragments as are known in the art, recombinant antibody fragments (such as sFv fragments, dsFv fragments, bispecific sFv fragments, bispecific dsFv fragments, F(ab)'₂ fragments, single chain Fv proteins ("scFv"), disulfide stabilized Fv proteins ("dsFv"), diabodies, and triabodies (as are known in the art), and camelid antibodies (see, for example, U.S. Pat. Nos. 6,015,695; 6,005,079-5,874,541; 5,840,526; 5,800,988; and 5,759,808). In some embodiments, a detectable moiety that generates a detectable signal is attached, covalently or otherwise, to the antigen binding molecule. Examples of suitable second binding pair members include, but are not limited to anti-DNP, anti-biotin, anti-FITC, anti-DIG, anti-NP, anti-NCA, anti-DPQ, anti-BF, anti-HQ, anti-DABSYL, anti-Rot, anti-BD, anti-CDO, anti-TS, and anti-Podo antibodies that are conjugated to a detectable moiety that generates a detectable signal. In further embodiments, second member of the binding molecule system is an anti-hapten primary antibody that does not comprise a detectable moiety. In these embodiments, the third member of the binding molecule system is a secondary anti-antibody (such as a goat antimouse IgG antibody) that comprises a detectable moiety that generates a signal is utilized for generating a detectable signal.

The signal generating systems of the present disclosure comprise a signal generating moiety. In preferred embodiments, the signal generating moiety can be detected by any known or yet to be a discovered mechanism including absorption, emission and/or scattering of a photon (including radio frequency, microwave frequency, infrared frequency, visible frequency and ultra-violet frequency photons). Signal-generating moieties include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), and paramagnetic and magnetic molecules or materials.

In some preferred embodiments, the signal generating moiety is an enzyme. Suitable enzymes include, but are not limited to, luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRPO), alkaline phosphatase, beta-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed J. Langone & H. Van Vunakis), Academic press, New York, 73:147-166 (1981). Where the detectable label includes an enzyme, a chromogen, fluorogenic compound, or luminogenic compound can be used in combination with the enzyme to generate a detectable signal (numerous of such compounds are commercially available, for example, from Invitrogen Corporation, Eugene Oreg.). Particular examples of chromogenic compounds include diaminobenzidine (DAB), 4-nitrophenylphospate (pNPP), fast red, bromochloroindolyl phosphate (BCIP), nitro blue tetrazolium (NBT), BCIP/NBT, fast red, AP Orange, AP blue, tetramethylbenzidine (TMB), 2,2'-azino-di-[3-ethylbenzothiazoline sulphonate] (ABTS), o-dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-.beta.-D-galactopyranoside (MU-Gal), p-nitrophenyl-α-D-galactopyranoside (PNP), 5-bromo-4-chloro-3-indolyl-β-D-glucuronide (X-Gluc), 3-amino-9-ethyl carbazol (AEC), fuchsin, iodonitrotetrazolium (INT), tetrazolium blue and tetrazolium violet.

Alternatively, an enzyme can be used in a metallographic detection scheme. For example, SISH procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate. (See, for example, U.S. Patent Application Publication No. 2005/0100976, PCT Publication No. 2005/003777 and U.S. Patent Application Publication No. 2004/0265922). Metallographic detection methods include using an oxido-reductase enzyme (such as horseradish peroxidase) along with a water soluble metal ion, an oxidizing agent and a reducing agent, again to for form a detectable precipitate. (See, for example, U.S. Pat. No. 6,670,113).

Particular examples of signal-generating moieties useful in the present invention include fluorescent molecules (or fluorochromes). Numerous fluorochromes are known to those of skill in the art, and can be selected, for example from Invitrogen, e.g., see, The Handbook--A Guide to Fluorescent Probes and Labeling Technologies, Invitrogen Detection Technologies, Molecular Probes, Eugene, Oreg.). Examples of particular fluorophores that can be attached (for example, chemically conjugated) to a nucleic acid molecule or protein such as an antigen binding molecule include, but are not limited to, 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumaran 151); cyanosine; 4',6-diaminidino-2-phenylindole (DAPI); 5',5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'S'-dichloro-6-carboxyfluorescein (JOE), fluorescein, fluorescein isothiocyanate (FITC), and QFITC (XRITC); 2',7'-difluorofluorescein (OREGON GREEN™); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron™ Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Other suitable fluorophores include thiol-reactive europium chelates which emit at approximately 617 nm (Heyduk and Heyduk, Analyt. Biochem. 248:216-27, 1997; J. Biol. Chem. 274:3315-22, 1999), as well as GFP, Lissamine, diethylaminocoumarin, fluorescein chlorotriazinyl, naphthofluorescein, 4,7-dichlororhodamine and xanthene (as described in U.S. Pat. No. 5,800,996 to Lee et al.) and derivatives thereof. Other fluorophores known to those skilled in the art can also be used, for example those available from Invitrogen Detection Technologies, Molecular Probes (Eugene, Oreg.) and including the ALEXA FLUOR™ series of dyes (for example, as described in U.S. Pat. Nos. 5,696,157, 6,130,101 and 6,716,979), the BODIPY series of dyes (dipyrrometheneboron difluoride dyes, for example as described in U.S. Pat. Nos. 4,774,339, 5,187,288, 5,248,782, 5,274,113, 5,338,854, 5,451,663 and 5,433,896), Cascade Blue (an amine reactive derivative of the sulfonated pyrene described in U.S. Pat. No. 5,132,432) and Marina Blue (U.S. Pat. No. 5,830,912).

In addition to the fluorochromes described above, a fluorescent label can be a fluorescent nanoparticle, such as a semiconductor nanocrystal, e.g., a QUANTUM DOT™ (obtained, for example, from QuantumDot Corp, Invitrogen Nanocrystal Technologies, Eugene, Oreg.; see also, U.S. Pat. Nos. 6,815,064, 6,682,596 and 6,649,138). Semiconductor nanocrystals are microscopic particles having size-dependent optical and/or electrical properties. When semiconductor nanocrystals are illuminated with a primary energy source, a secondary emission of energy occurs of a frequency that corresponds to the bandgap of the semiconductor material used in the semiconductor nanocrystal. This emission can be detected as colored light of a specific wavelength or fluorescence. Semiconductor nanocrystals with different spectral characteristics are described in e.g., U.S. Pat. No. 6,602,671. Semiconductor nanocrystals that can be coupled to a variety of biological molecules (including dNTPs and/or nucleic acids) or substrates by techniques described in, for example, Bruchez et. al. (1998) Science 281:2013-6, Chan et al. (1998) Science 281:2016-8, and U.S. Pat. No. 6,274,323.

Formation of semiconductor nanocrystals of various compositions are disclosed in, e.g., U.S. Pat. Nos. 6,927,069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Publication No. 2003/0165951 as well as PCT Publication No. 99/26299 (published May 27, 1999). Separate populations of semiconductor nanocrystals can be produced that are identifiable based on their different spectral characteristics. For example, semiconductor nanocrystals can be produced that emit light of different colors based on their composition, size or size and composition. For example, quantum dots that emit light at different wavelengths based on size (565 nm, 655 nm, 705 nm, or 800 nm emission wavelengths), which are suitable as fluorescent labels in the probes disclosed herein are available from Invitrogen.

Additional signal-generating moieties include, for example, radioisotopes (such as ³H, ³⁵S and ³²P), metal chelates such as DOTA and DPTA chelates of radioactive or paramagnetic metal ions like Gd³⁺, and liposomes.

In some embodiments, the signal-generating moiety is a fluorescent protein. Fluorescent proteins also can be used as a carrier, or can be coupled to a carrier, to facilitate visualization. For example, green fluorescent protein (GFP) was originally isolated from the light-emitting organ of the jellyfish *Aequorea victoria.* Chimeric GFP fusions can be expressed in situ by gene transfer into cells, and can be localized to particular sites within the cell by appropriate targeting signals. Spectral variants with blue, cyan and yellowish-green emissions have been successfully generated from the Aequorea GFP, but none exhibit emission maxima longer than 529 nm. GFP-like proteins have been isolated from Anthozoa (coral animals) that significantly expanded the range of colors available for biological applications. The family of 'GFP-like proteins' deposited in sequence databases now includes approximately 30 significantly different members. Fluorescent proteins refers to proteins that can become spontaneously fluorescent through the autocatalytic synthesis of a chromophore. Proteins that fluoresce at red or far-red wavelengths (red fluorescent proteins or RFPs) are known. RFPs can be used in combination with other fluorescent proteins that fluoresce at shorter wavelengths for both multicolor labeling and fluorescence resonance energy transfer (FRET) experiments. Commercially available RFPs are derived from two wild-type GFP-like proteins. DsRed (drFP583) has excitation and emission maxima at 558 nm and 583 nm, respectively. A far-red fluorescent protein was generated by mutagenesis of a chromoprotein that absorbs at 571 nm. HcRed1 (Clontech) has excitation and emission maxima at 588 nm and 618 nm, respectively. The fluorescent protein that emits fluorescence at the longest wavelength (without any mutations being introduced) is eqFP611, cloned from the sea anemone *Entacmaea quadricolor.* This protein absorbs at 559 nm and emits at 611 nm.

### 2. Immunohistochemistry

The present disclosure also describes concurrent detection of two or more forms of EGFR (e.g. wild-type, mutations of EGFR (e.g. Exon 19 del, L858R, etc.). In some embodiments, two or more (e.g. 2, 3, 4, 5, etc.) mutant forms of EGFR are detected with reagents (e.g. antibodies) of the present invention. In some embodiments, multiple forms of EGFR are detected in cells, tissue, tumor, cell culture, a sample, etc. In some embodiments, multiple forms of EGFR are detected in a tissue sample (e.g. section, core, biopsy, etc.) from a subject suspected of having cancer, a subject diagnosed with cancer, and/or a subject suffering from cancer. In some embodiments, multiple mutant forms of EGFR are detected, identified, and/or quantified in a sample (e.g. tissue sample). In some embodiments, the present invention comprises the concurrent detection of EGFR Exon 19 del and EGFR L858R.

In some embodiments, immunohistochemistry is performed on a tissue sample to detect gene copy number of a gene of interest (e.g. EGFR). Immunohistochemical staining of tissue sections has been shown to be a reliable method of assessing alteration of proteins in a heterogeneous tissue. Immunohistochemistry (IHC) techniques utilize an antibody to probe and visualize cellular antigens *in situ,* generally by chromagenic or fluorescent methods. This technique excels because it avoids the unwanted effects of disaggregation and allows for evaluation of individual cells in the context of morphology. In addition, the target protein is not altered by the freezing process.

In some embodiments, IHC is performed via a direct or indirect assay. In a direct IHC assay, binding of antibody to the target antigen is determined directly. This direct assay uses a labeled reagent (e.g. stain, fluorescent tag, enzyme-labeled primary antibody, etc.), which can be visualized without further antibody interaction. In a typical indirect assay, unconjugated primary antibody binds to the antigen and then a labeled secondary antibody binds to the primary antibody. In some embodiments, the secondary antibody is conjugated to a dye or an enzymatic label. In some embodiments, a chromagenic or fluorogenic substrate is added to provide visualization of the antigen. In some embodiments, indirect assays provide signal amplification through the interaction of several secondary antibodies with different epitopes on the primary antibody.

In some embodiments, following an optional blocking step, a tissue sample, section, or core is exposed to primary antibody for a sufficient period of time and under suitable conditions such that the primary antibody binds to the target protein antigen in the sample. Exemplary conditions appropriate for achieving binding are provided herein and understood by one in the art. In some embodiments, the extent of binding of antibody to the sample is determined by using any one of the detectable labels and techniques discussed above. In some embodiments, detection requires microscopy, fluorometry, and/or additional detection methods. In some embodiments, samples are mounted and coverslipped in preparation for detection.

In some embodiments, detection of multiple forms of EGFR with antibodies is performed concurrently with, and/or in conjunction with, one or more additional assays and/or detection techniques. The present invention may comprise, but is not limited to detection of multiple forms of EGFR by immunohistochemistry (e.g. antibody detection), detection and/or quantification of EGFR by *in situ* hybridization (e.g. chromogenic *in situ* hybridization (CISH), fluorescence *in situ* hybridization (FISH), *silver in situ* hybridization (SISH) etc.), PCR amplification of one or more forms of EGFR, etc.

### 3. In situ hybridization

The present disclosure also describes compositions and methods for the detection and/or quantification of genetic sequences associated with EGFR (e.g. all forms of EGFR, wild-type EGFR, mutant EGFR (e.g. EGFR Exon 19 del and EGFR L858R, etc.). In some embodiments, reagents of the present invention for detection and/or quantification of EGFR related sequences include, but are not limited to proteins (e.g. antigen-binding proteins, immunoglobulins, polyclonal antibodies, monoclonal antibodies, chimeric antibodies, single chain antibodies, and humanized antibodies, Fab fragments, etc.), nucleic acids (e.g. oligonucleotide probes, label-oligonucleotide conjugate probes, etc.), small molecules, etc. In some embodiments, nucleic acid probes are used to detect and/or quantify EGFR related sequences in cells, cell culture, tissues, tissue samples, tumor samples, etc. In some embodiments, labeled oligonucleotide probes (e.g. chromophore-labeled probes, fluorophore-labeled probes, radioactively-labeled probes, enzyme-linked probes, etc.) provide identification, detection, and/or quantification of genetic sequences associated with EGFR. In some embodiments, *in situ* hybridization (e.g. FISH, CISH, etc.) provides detection and/or quantification of EGFR related sequences. In some embodiments, chromogenic *in situ* hybridization provides detection and/or quantification of EGFR sequences (e.g. all EGFR, mutant forms of EGFR (e.g. EGFR Exon 19 del and EGFR L858R, etc.), wild-type EGFR, etc.).

In some embodiments, detection and/or quantification of EGFR related sequences yields an accurate and/or estimate of the EGFR gene copy number. In some embodiments, detection, quantification, and/or estimation of EFGR gene copy number provides an assessment of gene copy number gain (e.g. change in gene copy number). In some embodiments, comparison of measured gene copy to a standard value, to a measured standard or reference, and/or to another sample from the same subject (e.g. tissue from a different location, tissue sample taken at a different time) provides assessment of the change in gene copy number (e.g. EGFR gene copy number gain). In some embodiments, increase in gene copy number is the result of gene amplification in a tissue.

In some embodiments, EGFR gene amplification (e.g. increase in EGFR gene copy number) and mutation of EGFR (e.g. EGFR Exon 19 del, EGFR L858R, etc.) are linked and/or associated (Okabe et al. Cancer Res 2007; 67: (5)). In some embodiments, EGFR mutations and EGFR copy number is indicative of clinical outcome in cancer patients (NSCLC patients). In some embodiments, EGFR mutations and increased copy numbers are significantly associated with positive clinical outcomes in patients (e.g. cancer patients, breast cancer patient, lung cancer patients, NSCLC patients, patients treated with small molecule receptor tyrosine kinase inhibitors (e.g. gefitinib)) (Takano et al. J Clin Oncol 23:6829-6837). In some embodiments, high levels of EGFR gene expression corresponds with EGFR gene amplification (e.g. high EGFR gene copy number, e.g. EGFR copy number gain, etc.) (Wong et al. Proc. Natl. Acad. Sci. USA. Vol. 84, pp. 6899-6903). In some embodiments, EGFR gene amplification corresponds with EGFR overexpression (Bhargava et al. Modern Pathology (2005) 18, 1027-1033).

In some embodiments, the present invention provides detection of EGFR gene copy number through the use of *in situ* hybridization techniques (e.g. chromogenic or *silver in situ* hybridization). In some embodiments, standard *in situ* hybridization techniques are applied. Standard *in situ* hybridization techniques are described in the art (e.g. Gall and Pardue, Methods in Enzymology, Vol. 21, pgs. 470-480 (1981); Henderson, International Review of Cytology, Vol. 76, pgs. 1-46 (1982); Angerer, et al., Genetic Engineering: Principles and Methods, Setlow and Hollaender, Eds., Vol. 7, pgs. 43-65 (Plenum Press, New York, 1985).

In some embodiments*, in situ* hybridization comprises the following major steps: (1) fixation of tissue or biological structure to be examined, (2) prehybridization treatment of the biological sample to increase accessibility of target DNA, and to reduce nonspecific binding, (3) hybridization probe to the nucleic acid in the biological sample; (4) post-hybridization washes to remove probe not bound in specific hybrids, and (5) detection of the hybridized probes. These steps, the reagents used, and their conditions of use vary in different embodiments of the present invention.

In some embodiments, the present disclosure provides oligonucleotide probes configured to hybridize to regions of the EGFR gene. In some embodiments, EGFR probes are configured to bind to the EGFR gene regions under low-stringency conditions. In some embodiments, EGFR probes are configured to bind to the EGFR gene regions under moderate-stringency conditions, but not low-stringency conditions. In some embodiments, EGFR probes are configured to bind to the EGFR gene regions only under high-stringency conditions.

In some embodiments, *in situ* hybridization comprises chromogenic *in situ* hybridization (CISH), *silver in situ* hybridization (SISH) or fluorescence *in situ* hybridization (FISH). In some embodiments, CISH is performed for the EGFR gene region. For example, tissue sections are incubated at 55 C overnight. Slides are deparaffinized in xylene and graded ethanols. Heat pretreatment is carried out in the pretreatment buffer (Zymed Laboratories Inc.) at 98-100C for 15 min. The tissue is digested with pepsin for 10 min at room temperature. After application of EGFR probe, the slides are coverslipped and edges sealed with rubber cement. The slides are heated at 95C for 5 min followed by overnight incubation at 37C using a moisturized chamber. Post-hybridization wash is performed and followed by immunodetection using a CISH polymer detection kit (e.g. from Zymed Laboratories Inc.). The CISH signals are counted using a light microscope with a x40 objective. In some embodiments, a tumor is interpreted as positive for gene amplification when the average number of gene copies was 45 per nucleus. One of skill in the art is capable of adjusting parameters to optimize *in situ* hybridization.

Chromogenic *in situ* hybridization (CISH) is a technique that allows *in situ* hybridization methods to be performed and detected with a bright-field microscope, instead of a fluorescence microscope as required for FISH. While FISH requires a fluorescence microscopes equipped with high-quality 60X or 100X oil immersion objectives and multi-band-pass fluorescence filters (not used in most routine diagnostic laboratories), CISH allows detection with standard light (bright-field) microscopes (which are generally used in diagnostic laboratories). CISH does not generally fade, allowing the tissue samples to be archived and reviewed later. Histological detail is better appreciated with bright-field detection, which is possible with CISH detection. Large regions of tissue section can be scanned rapidly after CISH counterstaining since morphological detail is readily apparent using low power objectives (e.g. 10X and 20X), while FISH detection generally requires substantially higher magnification (thus reducing the field of view).

General chromogenic/colorimetric *in situ* hybridization methods are described in WO0026415 to Fletcher et al. and US Pat Pub. No. 20030017491. Particular reagents and steps for performing CISH on formalin-fixed, paraffin-embedded (FFPE) tissue samples, as well as cell sample/metaphase chromosome samples are described in WO0026415, US Pat Pub. No. 20030017491, and the section presented below. Importantly the description detailed below provides exemplary methods, procedures, and reagents, and is not to be construed as limiting the present invention.

### 4. Diagnostics and Kits

In some embodiments, compositions and methods of the present invention are applied to tissues, cells, biopsy tissue, tissue samples (e.g. core, section, etc.), etc. from subjects suspected of having cancer, diagnosed with cancer, and/or suffering from cancer. In some embodiments, tissue biopsies are sectioned and protein detected using, for example, immunofluorescence, immunohistochemistry, *in situ* hybridization (e.g. CISH, FISH, etc.).

In some embodiments, compositions and methods of the present invention find use in accessing and treating cancers including, but not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, non-small cell lung cancer, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

In some embodiments, the present invention provides kits as defined in claim 16 for the detection, characterization, and/or quantification of EGFR mutant forms.

### EXPERIMENTAL

The following section provides exemplary embodiments of the present invention, and should not be considered to be limiting of its scope with regard to alternative embodiments that are not explicitly described herein.

### Example 1

### Compositions and Methods for Gene-Protein Assay

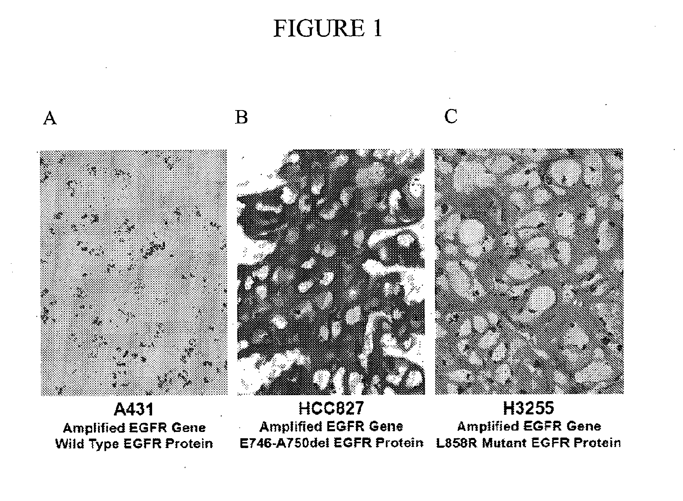

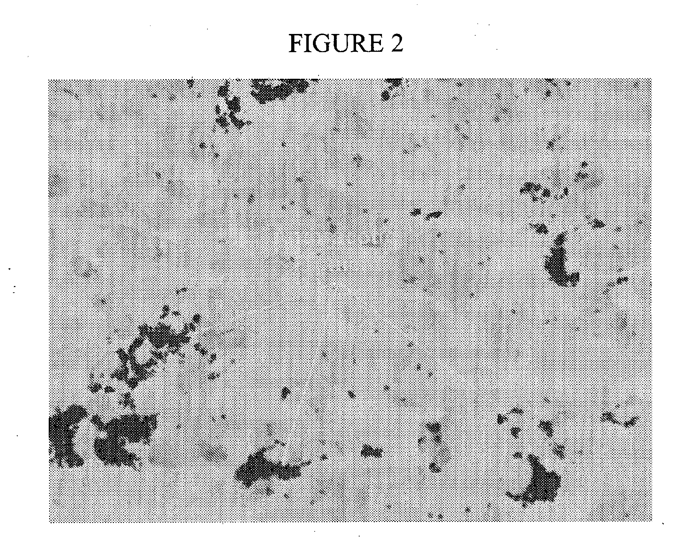

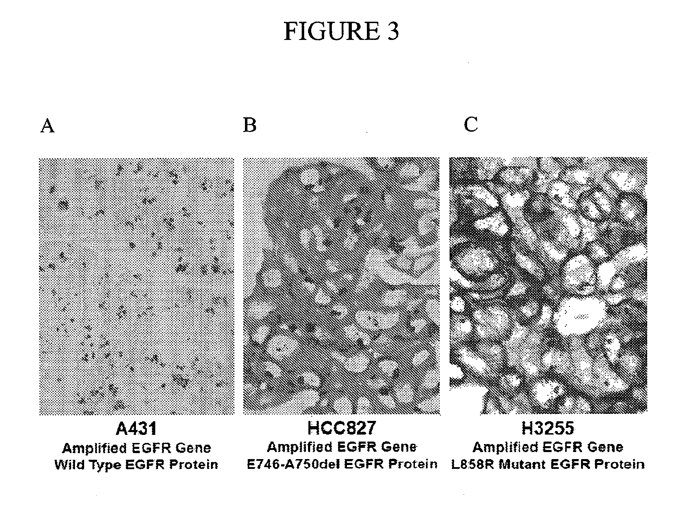

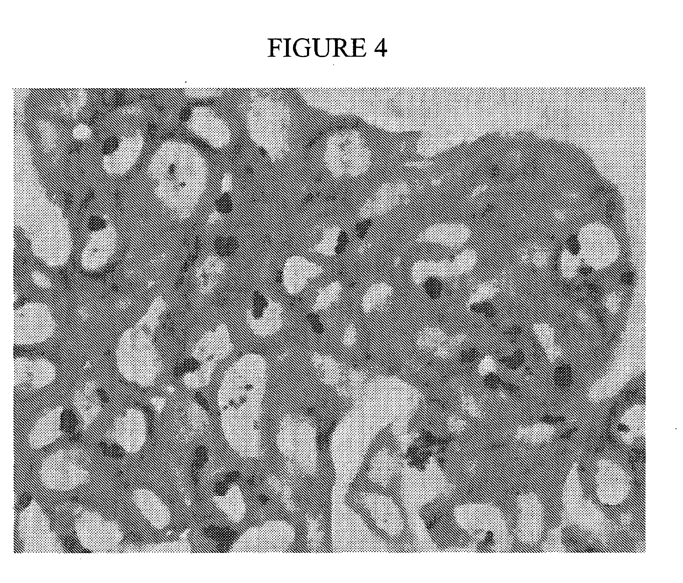

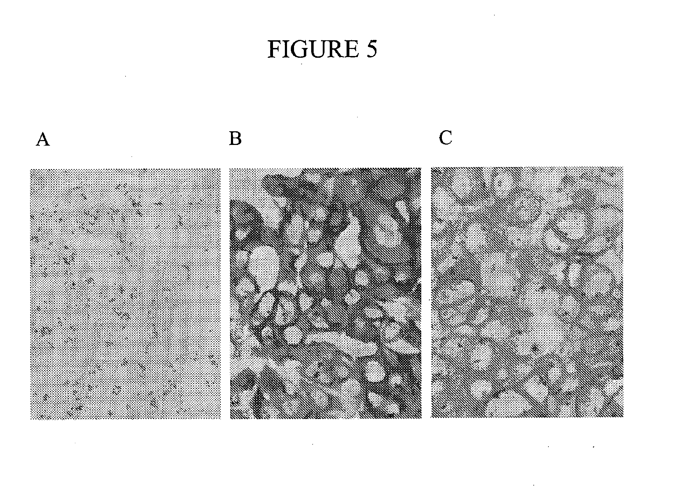

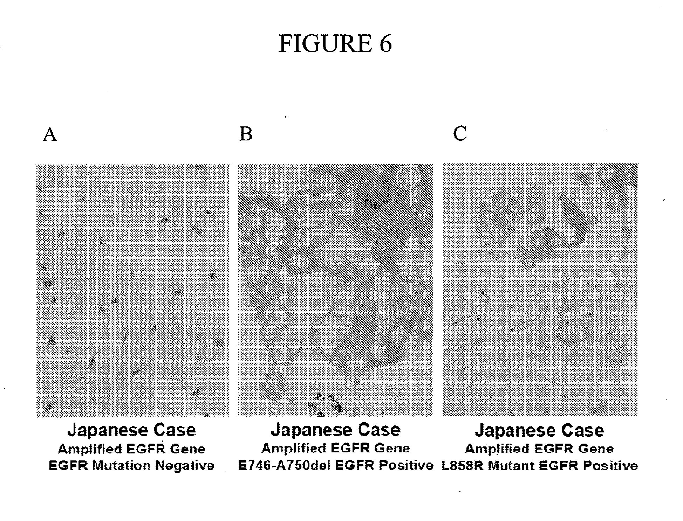

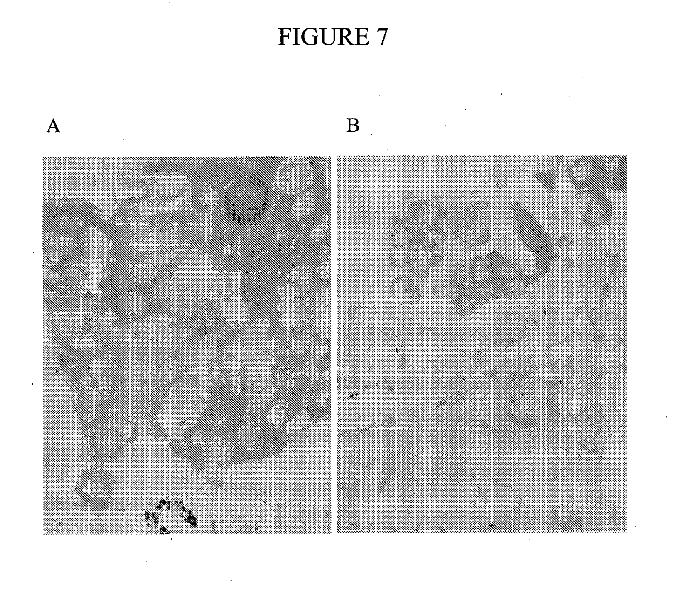

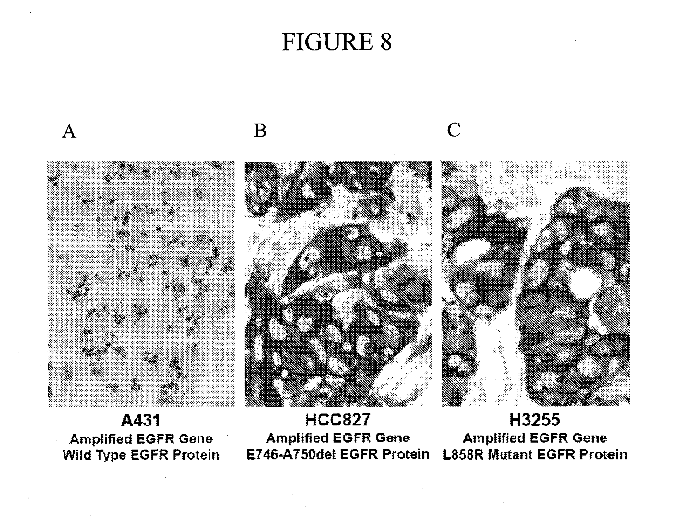

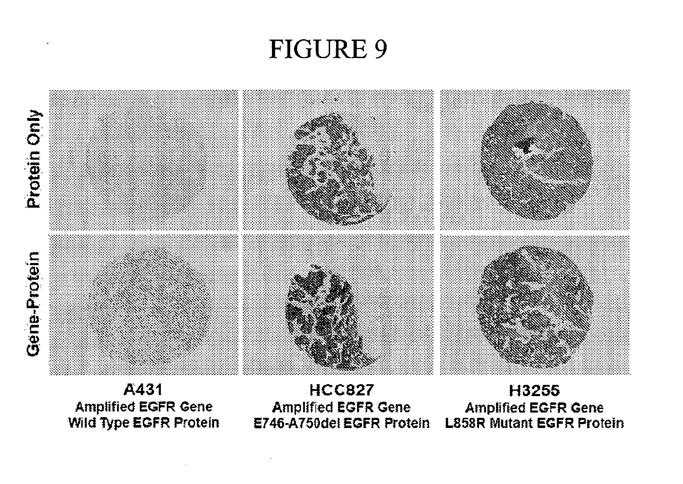

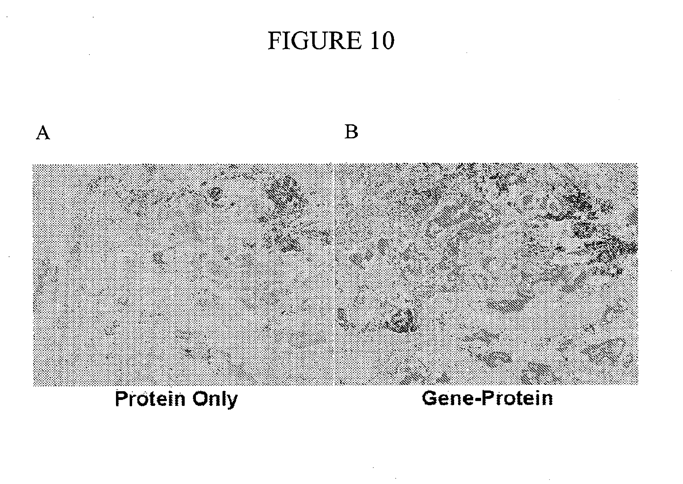

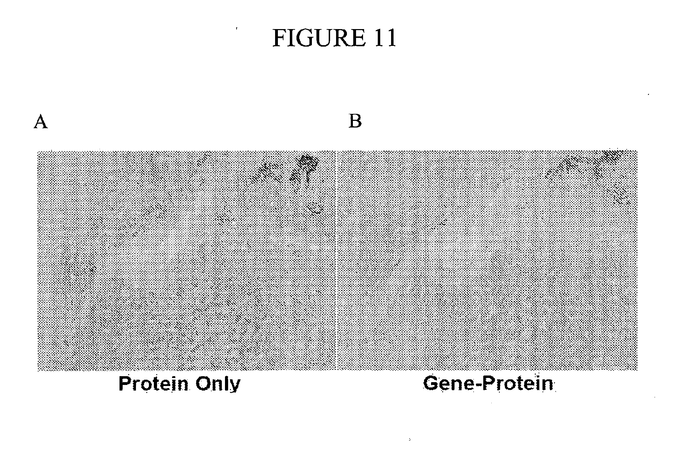

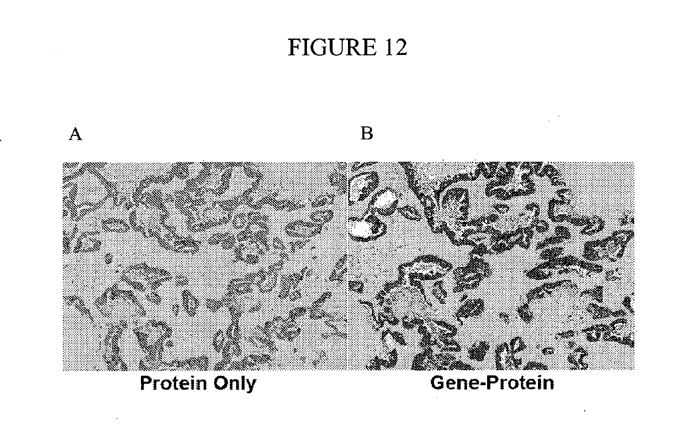

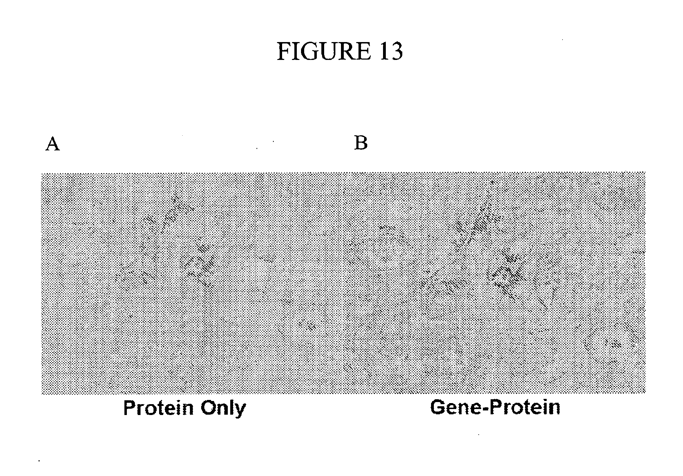

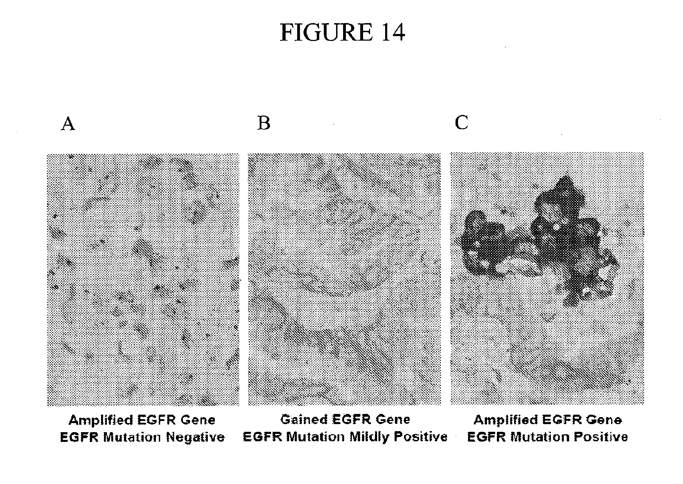

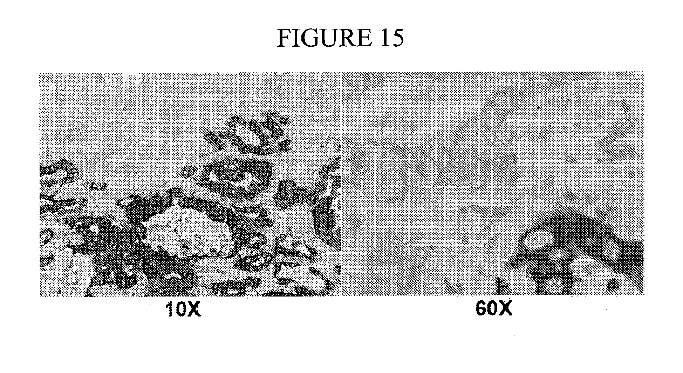

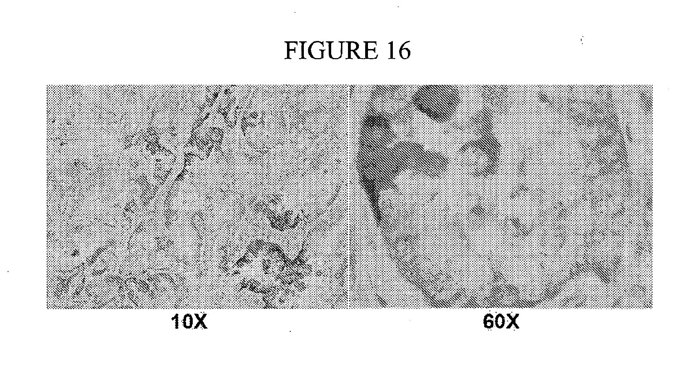

The following section provides exemplary embodiments of the present invention, and should not be considered to be limiting of its scope with regard to alternative embodiments that are not explicitly described herein. Figures 1-16 provide examples of tissues subjected to the compositions and methods described herein.

*Anti-EGFR Antibodies.* Rabbit monoclonal L858R mutant specific anti-EGFR (clone 43B2) and E746-A750del specific anti-EGFR (clone 6B6) antibodies were obtained from Cell Signaling Technology, Inc., Danvers, MA. Antibodies were diluted in SIGNALSTAIN Antibody Diluent (Cell Signaling Technology) at 1:30.

*EGFR Probe.* Nick-translated, DNP-labeled INFORM EGFR probe (catalog # 980-4343) was obtained from Ventana Medical Systems, Inc., Tucson, AZ.

*Xenograft Tumor Tissue Microarray.* The following xenograft tumors were fixed in 10% neutral buffered formalin and embedded in paraffin: H460 (monosomy EGFR gene, wild type EGFR protein), H3255 (amplified EGFR gene, L858R EGFR protein), A549 (trisomy EGFR gene, wild type EGFR protein), RVH6849 (amplified EGFR gene, wild type EGFR protein), HCC827 (amplified EGFR gene, E746-A750del EGFR protein), and A431 (amplified EGFR gene, wild type EGFR protein). Tissue cores were prepared from each xenograft tumors and re-embedded in paraffin as a tissue microarray with 6 xenograft tumor tissue cores.

*EGFR Mutation Detection Protocols.* Using a dual color gene-protein method, EGFR immunohistochemistry (IHC) detection was conducted prior to EGFR *in situ* hybridization (ISH). The slides were deparaffinized using EZ Prep (catalog # 950-102) on BenchMark XT. Then, the slides were subjected for heat pretreatment using Cell Conditioning 1 (catalog # 950-124) prior to antibody incubation. Mixture of L858R mutant specific antibody and E746-A750del antibody was dispensed onto the slides and incubated for 1 hour at 37°C. After washing the slides with Reaction Buffer (catalog # 950-300), alkaline phosphatase (AP) conjugated goat anti-rabbit antibody was applied onto the slides and incubated for 16 minutes at 37°C. Color development was performed with naphthol and fast blue. Then, the slides were processed for heat-pretreatment with Reaction Buffer and digested with ISH Protease 3 (catalog # 780-4149). EGFR probe was applied to tissue sections and hybridized for 5 hours after probe and target co-denaturation step. Stringency wash steps were conducted with SSC (catalog # 950-110) and tissue sections were incubated with rabbit monoclonal anti-DNP antibody (catalog # 780-4335). Then, the slides were incubated with AP-conjugated anti-rabbit antibody and color development was conducted with naphthol and fast red (based on a Ventana provisional patent application). The tissue sections were counterstained with Hematoxylin II (catalog # 790-2208) and Bluing Reagent (catalog # 760-2037).

Unsing a triple color gene-protein method, EGFR immunohistochemistry (IHC) detection was conducted prior to EGFR *in situ* hybridization (ISH). The slides were deparaffinized using EZ Prep (catalog # 950-102) on BenchMark XT. Then, the slides were subjected for heat pretreatment using Cell Conditioning 1 (catalog # 950-124) prior to antibody incubation. E746-A750del antibody was dispensed onto the slides and incubated for 1 hour at 37°C. After washing the slides with Reaction Buffer, AP conjugated goat anti-rabbit antibody was applied onto the slides and incubated for 16 minutes at 37°C. Color development was performed with naphthol and fast blue. Then, the slides were heated for inactivating reagents for the first IHC detection. L858R mutant specific antibody was dispensed onto the slides and incubated for 1 hour at 37°C. After washing the slides with Reaction Buffer, AP conjugated goat anti-rabbit antibody was applied onto the slides and incubated for 16 minutes at 37°C. Color development was performed with naphthol and fast red. Then, the slides were processed for heat-pretreatment with Reaction Buffer and digested with ISH Protease 3 (catalog # 780-4149). EGFR probe was applied to tissue sections and hybridized for 5 hours after probe and target co-denaturation step. Stringency wash steps were conducted with SSC (catalog # 950-110) and tissue sections were incubated with rabbit monoclonal anti-DNP antibody (catalog # 780-4335). Then, the slides were incubated with horseradish peroxidase-conjugated anti-rabbit antibody and color development was conducted with ultraView SISH kit (catalog # 780-002). The tissue sections were counterstained with Hematoxylin II (catalog # 790-2208) and Bluing Reagent (catalog # 760-2037).

## Claims

1. A method for assessing the EGFR status of a tissue sample comprising:
processing said tissue sample with reagents to produce distinguishable signals corresponding to the presence or absence of a L858R EGFR mutation, an exon 19 deletion EGFR mutation and EGFR gene amplification; and
simultaneously visualizing said distinguishable signals.

2. The method of claim 1, wherein said tissue sample is obtained from a subject suspected of having cancer, a subject diagnosed with cancer, or a subject suffering from cancer.

3. The method of claim 1, wherein said processing comprises contacting said sample with antigen binding molecules specific for EGFR molecules comprising the L858R and/or exon 19 deletion mutation.

4. The method of claim 3, wherein said processing further comprises contacting said sample with nucleic acid probes specific for the EGFR gene.

5. The method of claim 4, wherein said processing further comprises contacting said antigen binding molecules specific for EGFR molecules comprising the L858R and/or exon 19 deletion mutation and said nucleic acid probe specific for the EGFR gene with reagents that produce a detectable signal corresponding to the presence or absence of said EGFR L858R and/or exon 19 deletion mutation and the presence or absence of EGFR gene amplification.

6. The method of claim 5, wherein said antigen binding molecules specific for EGFR molecules comprising the L858R and/or exon 19 deletion mutation and said nucleic acid probe specific for the EGFR gene comprise a signal generating moiety.

7. The method of claim 5, wherein said antigen binding molecules specific for EGFR molecules comprising the L858R and/or exon 19 deletion mutation and said nucleic acid probe specific for the EGFR are detected with signal generating systems.

8. The method of Claim 7, wherein said signal generating systems comprise reagents for the differential detection of said antigen binding molecules specific for EGFR molecules comprising the L858R and/or exon 19 deletion mutation and said nucleic acid probe specific for the EGFR.

9. The method of Claim 8, wherein said signal generating system comprises reagents for generating different colorimetric signals for each of the said antigen binding molecules specific for EGFR molecules comprising the L858R and/or exon 19 deletion mutation and said nucleic acid probe specific for the EGFR.

10. The method of Claim 9, wherein said reagents for generating different colorimetric signals are selected from the group consisting of silver, fast red, fast blue, fast gold, DAB, AP orange, and AP blue.

11. The method of Claim 9, wherein said reagents for generating different colorimetric signals comprise enzymatically labeled reagents.

12. The method of Claim 11, wherein the enzyme labels of said enzymatically labeled reagents are selected from the group consisting of horseradish peroxidase, alkaline phosphatase, acid phosphatase, glucose oxidase, β-galactosidase, β-glucouronidase and β-lactamase.

13. The method of claim 1, further comprising:
evaluating changes in EGFR gene copy number and the presence or absence of EGFR mutations.

14. The method of Claim 13, further comprising using said evaluation to make a diagnosis of prognosis for the patient.

15. The method of Claim 13, further comprising using said evaluation to determine a therapeutic treatment.

16. A kit comprising:
a first antigen binding molecule specific for EGFR molecules comprising a L858R mutation;
a second antigen binding molecule specific for EGFR molecules comprising an exon 19 deletion mutation;
a nucleic acid probe specific for EGFR;
and distinguishable signal-generating reagents specific for each of said first antigen binding molecule, said second antigen binding molecule, and said nucleic acid probe.

## Patentansprüche

1. Verfahren zum Beurteilen des EGFR-Zustands einer Gewebeprobe, das Folgendes umfasst:
Weiterverarbeiten der Gewebeprobe mit Reagenzien, um unterscheidbare Signale zu erzeugen, die dem Vorliegen oder Fehlen einer L858R-EGFR-Mutation, einer Exon-19-Deletion-EGFR-Mutation und einer EGRFEGFR-Genamplifikation entsprechen; und
Gleichzeitiges Sichtbarmachen dieser unterscheidbaren Signale.

2. Verfahren nach Anspruch 1, wobei die Gewebeprobe von einem Individuum, das unter Krebsverdacht steht, einem Individuum, das mit Krebs diagnostiziert worden ist oder einem Individuum, das an Krebs leidet, stammt.

3. Verfahren nach Anspruch 1, wobei das Weiterverarbeiten das Inkontaktbringen der Probe mit antigenbindenden Molekülen mit Spezifität für EGFR-Moleküle umfassend die L858R- und/oder Exon-19-Deletions-Mutation umfasst.

4. Verfahren nach Anspruch 3, wobei das Weiterverarbeiten weiterhin das Inkontaktbringen der Probe mit Nukleinsäuresonden mit Spezifität für das EGFR-Gen umfasst.

5. Verfahren nach Anspruch 4, wobei das Weiterverar-beiten weiterhin das Inkontaktbringen der antigenbindenden Moleküle mit Spezifität für EGFR-Moleküle umfassend die L858R- und/oder Exon-19-Deletions-Mutation und der Nukleinsäuresonde mit Spezifität für das EGFR-Gen mit Reagenzien, die ein nachweisbares Signal erzeugen, das dem Vorliegen oder Fehlen der EGFR-L858R- und/oder Exon-19-Deletions-Mutation und dem Vorliegen oder Fehlen einer EGFR-Genamplifikation entspricht, umfasst.

6. Verfahren nach Anspruch 5, wobei die antigenbindenden Moleküle mit Spezifität für EGFR-Moleküle umfassend die L858R- und/oder Exon-19-Deletions-Mutation und die Nukleinsäuresonde mit Spezifität für das EGFR-Gen einen signalerzeugenden Molekülrest umfassen.

7. Verfahren nach Anspruch 5, wobei die antigenbindenden Moleküle mit Spezifität für EGFR-Moleküle umfassend die L858R- und/oder Exon-19-Deletions-Mutation und die Nukleinsäuresonde mit Spezifität für das EGFR-Gen mit signalerzeugenden Systemen nachgewiesen werden.

8. Verfahren nach Anspruch 7, wobei die signalerzeugenden Systeme Reagenzien für den differenziellen Nachweis der antigenbindenden Moleküle mit Spezifität für EGFR-Moleküle umfassend die L858Rund/oder Exon-19-Deletions-Mutation und die Nukleinsäuresonde mit Spezifität für das EGFR-Gen umfassen.

9. Verfahren nach Anspruch 8, wobei das signalerzeugende System Reagenzien zum Erzeugen von unterschiedlichen kolorimetrischen Signalen für jedes der antigenbindenden Moleküle mit Spezifität für EGFR-Moleküle umfassend die L858R- und/oder Exon-19-Deletions-Mutation und die Nukleinsäuresonde mit Spezifität für das EGFR-Gen umfasst.

10. Verfahren nach Anspruch 9, wobei die Reagenzien zum Erzeugen von unterschiedlichen kolorimetrischen Signalen aus der Gruppe bestehend aus Silber, Fast Red, Fast Blue, Fast Gold, DAB, AP Orange und AP Blue ausgewählt sind.

11. Verfahren nach Anspruch 9, wobei die Reagenzien zum Erzeugen von unterschiedlichen kolorimetrischen Signalen enzymmarkierte Reagenzien umfassen.

12. Verfahren nach Anspruch 11, wobei die Enzymmarker der enzymmarkierten Reagenzien aus der Gruppe bestehend aus Meerrettichperoxidase, alkalischer Phosphatase, saurer Phosphatase, Glucoseoxidase, β-Galactosidase, β-Glucouronidase und β-Lactamase ausgewählt sind.

13. Verfahren nach Anspruch 1, das weiterhin Folgendes umfasst:
Auswerten von Veränderungen bezüglich der EGFR-Genkopienzahl und des Vorliegens oder Fehlens von EGFR-Mutationen.

14. Verfahren nach Anspruch 13, das weiterhin Folgendes umfasst: Verwenden dieser Auswertung zum Erstellen einer Diagnose für die Prognose des Patienten.

15. Verfahren nach Anspruch 13, das weiterhin Folgendes umfasst: Verwenden dieser Auswertung zum Festlegen einer therapeutischen Behandlung.

16. Kit, das Folgendes umfasst:
ein erstes antigenbindendes Molekül mit Spezifität für EGFR-Moleküle umfassend eine L858R-Mutation;
ein zweites antigenbindendes Molekül mit Spezifität für EGFR-Moleküle umfassend eine Exon-10-Deletions-Mutation;
eine Nukleinsäuresonde mit Spezifität für EGFR;
und unterscheidbare signalerzeugende Reagenzien mit Spezifität für jedes der Genannten: erstes antigenbindendes Molekül, zweites antigenbindendes Molekül und Nukleinsäuresonde.

## Revendications

1. Procédé d'évaluation du statut EGFR d'un échantillon de tissu comprenant les étapes consistant à :
traiter ledit échantillon de tissu avec des produits réactifs afin de produire des signaux perceptibles correspondant à la présence ou à l'absence d'une mutation de type L858R de l'EGFR, une mutation de l'EGFR de type délétion dans l'exon 19 et une amplification génétique de l'EGFR ; et
visualiser de manière simultanée lesdits signaux perceptibles.

2. Procédé selon la revendication 1, dans lequel ledit échantillon de tissu est obtenu à partir d'un sujet suspecté de présenter un cancer, un sujet diagnostiqué avec un cancer, ou un sujet souffrant d'un cancer.

3. Procédé selon la revendication 1, dans lequel ladite étape de traitement comprend la mise en contact dudit échantillon avec des molécules de liaison à l'antigène spécifiques aux molécules EGFR comprenant la mutation de type L858R et/ou de type délétion dans l'exon 19.

4. Procédé selon la revendication 3, dans lequel ladite étape de traitement comprend en outre la mise en contact dudit échantillon avec des sondes nucléiques spécifiques au gène EGFR.

5. Procédé selon la revendication 4, dans lequel ladite étape de traitement comprend en outre la mise en contact desdites molécules de liaison à l'antigène spécifiques aux molécules EGFR comprenant la mutation de type L858R et/ou de type délétion dans l'exon 19 et ladite sonde nucléique spécifique au gène EGFR avec des produits réactifs qui produisent un signal détectable correspondant à la présence ou à l'absence de ladite mutation de l'EGFR de type L858R et/ou de type délétion dans l'exon 19 et la présence ou l'absence d'une amplification génétique de l'EGFR.

6. Procédé selon la revendication 5, dans lequel lesdites molécules de liaison à l'antigène spécifiques aux molécules EGFR comprenant la mutation de type L858R et/ou de type délétion dans l'exon 19 et ladite sonde nucléique spécifique au gène EGFR comprennent un groupement générateur de signal.

7. Procédé selon la revendication 5, dans lequel lesdites molécules de liaison à l'antigène spécifiques aux molécules EGFR comprenant la mutation de type L858R et/ou de type délétion dans l'exon 19 et ladite sonde nucléique spécifique à l'EGFR sont détectées à l'aide de systèmes générateurs de signal.

8. Procédé selon la revendication 7, dans lequel lesdits systèmes générateurs de signal comprennent des produits réactifs pour la détection différentielle desdites molécules de liaison à l'antigène spécifiques aux molécules EGFR comprenant la mutation de type L858R et/ou de type délétion dans l'exon 19 et ladite sonde nucléique spécifique à l'EGFR.

9. Procédé selon la revendication 8, dans lequel ledit système générateur de signal comprend des produits réactifs permettant de générer différents signaux colorimétriques pour chacune desdites molécules de liaison à l'antigène spécifiques aux molécules EGFR comprenant la mutation de type L858R et/ou de type délétion dans l'exon 19 et ladite sonde nucléique spécifique à l'EGFR.

10. Procédé selon la revendication 9, dans lequel lesdits produits réactifs permettant de générer différents signaux colorimétriques sont sélectionnés parmi le groupe constitué d'argent, rouge vif, bleu vif, or vif, DAB (3,3'-diaminobenzimidine), orange AP (phosphatase alcaline), et bleu AP.

11. Procédé selon la revendication 9, dans lequel lesdits produits réactifs permettant de générer différents signaux colorimétriques comprennent des produits réactifs marqués de manière enzymatique.

12. Procédé selon la revendication 11, dans lequel les enzymes marqueurs desdits produits réactifs marqués de manière enzymatique sont sélectionnés parmi le groupe constitué de la peroxydase de raifort, la phosphatase alcaline, la phosphatase acide, l'oxydase de glycose, la β-galactosidase, la β-gluco-uronidase et la β-lactamase.

13. Procédé selon la revendication 1, comprenant en outre l'étape consistant à :
évaluer des modifications du nombre de copies du gène EGFR et la présence ou l'absence de mutations de l'EGFR.

14. Procédé selon la revendication 13, comprenant en outre une étape consistant à utiliser ladite évaluation pour faire un diagnostic ou un pronostic pour le patient.

15. Procédé selon la revendication 13, comprenant en outre une étape consistant à utiliser ladite évaluation pour déterminer un traitement thérapeutique.

16. Trousse comprenant :
une première molécule de liaison à l'antigène spécifique aux molécules EGFR comprenant une mutation de type L858R ;
une seconde molécule de liaison à l'antigène spécifique aux molécules EGFR comprenant une mutation de type délétion dans l'exon 19 ;
une sonde nucléique spécifique à l'EGFR ;
et des produits réactifs générant un signal perceptible spécifique à chacune parmi ladite première molécule de liaison à l'antigène, ladite seconde molécule de liaison à l'antigène, et ladite sonde nucléique.
